# EUROPEAN PATENT APPLICATION

(11) **EP 4 060 022 A1**
(43) Date of publication of application: **21.09.2022**
(21) Application number: 20874547.1
(22) Date of filing: 08.10.2020
(51) Int. Cl.: C12N 5/071

(54) **TONSILAR ORGANOID PREPARATION METHOD AND USE THEREOF**

(30) Priority: 11.10.2019 KR 20190126284
(71) Applicant: Organoidsciences, Ltd., Jungwon-gu Seongnam-si, Gyeonggi-do 13230 (KR)
(72) Inventor: YOO, Jong Man, Seongnam-si, Gyeonggi-do 13540 (KR); LIM, Young Chang, Seoul 07651 (KR); KIM, Han Kyung, Seongnam-si, Gyeonggi-do 13505 (KR); KIM, Hye Ryeon, Seoul 02201 (KR)
(74) Representative: Rückerl, Florian
(86) International application number: PCT/KR2020/013714
(87) International publication number: WO 2021/071270

(57) **Abstract**

The present invention relates to a tonsillar organoid preparation method and use thereof, which provides a tonsillar organoid preparation medium comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM). This enables long-term subculture of tonsil organoids that show the same physiological activity as that of a real tonsil tissue.

## Description

### [Technical Field]

The present invention relates to a tonsillar organoid preparation method and use thereof.

### [Background Art]

Tonsils are a set of mucosa-associated lymphoid tissue at the gateway to the digestive and respiratory tracts known as Waldeyer's tonsillar ring, comprising a pharyngeal tonsil, two tubal tonsils, two palatine tonsils and lingual tonsils. Each tonsil consists of an epithelium overlying lymph follicles and serves as the first line of defense against ingested or inhaled pathogens such as bacteria or viruses. The crypts of the tonsil epithelium recognize the pathogens introduced through the mouth and transmit signals to stimulate immune responses by B cells, T cells, macrophages and dendritic cells. The tonsils are susceptible to bacterial and viral infections, which can lead to tonsillitis, enlarged tonsils and cancer of the pharynx when infected.

Previous studies have shown that bacterial and viral infections of the tonsils are a major cause of chronic tonsil disorder, and pharmacological, nutritional, and polymorphic factors are also considered as important causes of chronic tonsil disorder. However, the pathophysiology remains unclear and controversial. Therefore, there is no ex vivo model suitable for reproducing the human tonsils for this pathological study, and it is difficult to study the pathophysiology of tonsils because experimental animal models cannot be used.

Based on these problems, recent advances in organ 3D culture from human specimens or epithelial stem cells have made possible the generation of human epithelial organoids. This 3D culture model could represent human body physiology since the human epithelial organoids comprise structures differentiated and proliferated from all tissue-specific epithelial cell types and epithelial stem cells. So far, this method has been extensively used to generate epithelial tissue-related disease models and to study epithelial biology (see Korean patent no. 10-18719490000), but method for preparing tonsil organoids that maintain essential functions of tonsil epithelium, such as cellular composition and microstructure, are still lacking.

### [Technical Problem]

One aspect of the present invention provides a tonsillar organoid preparation medium (Tonsil Expansion Medium, TEM) additionally comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM).

In another embodiment, the tonsillar organoids preparation medium comprises a HGF, a FGF10, a FGF2, a FGF basic, a PGE2, an A83-01, a Nicotinamide, a Noggin, a R-spondin1, an antibiotic-antifungal agent, a glutamax, a B27, a SB202190, a penicillin, a streptomycin, an EGF and a HEPES.

In another embodiment, the HGF is comprised in a concentration of 20 to 80 ng/ml in the total tonsil organoid preparation medium composition.

In another embodiment, a tonsil organoid prepared by the tonsillar organoid preparation medium expresses genes of a CD44, a nerve growth factor receptor, a cytokeratin 5, a cytokeratin 13, an Integrin alpha 6, and an epithelial cell adhesion molecule.

In another embodiment, a tonsil organoid prepared by the tonsillar organoid preparation medium expresses a toll-like receptor (TLR) 2, 3, 4, and 6.

In another embodiment, a tonsillar organoid prepared by the tonsillar organoid preparation medium can be subcultured at least second generations.

Another aspect of the present invention provides a method for preparing tonsil organoid comprising: preparing a tonsillar organoid preparation medium additionally comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM), and culturing a tonsil organoid in the tonsillar organoid preparation medium.

Other objects and advantages of the present application will become more apparent from the following detailed description with the appended claims and figures. Contents not described in this specification can be sufficiently recognized and inferred by those skilled in the technical field or similar technical field of the present application, and thus are omitted.

### [Technical Solution]

One aspect of the present invention provides a tonsillar organoid preparation medium (Tonsil Expansion Medium, TEM) additionally comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM).

As used herein, the term "organoid" refers to a cell having a 3D three-dimensional structure, and refers to a organ-like model prepared through an artificial culture process that is not collected or acquired from animals. The origin of the cells constituting it is not limited. Unlike a 2D culture, a 3D cell culture allows cells to grow in all directions ex vivo, and thus the organoids can be used to develop therapeutic agents for diseases by similarly mimicking organs interacting in vivo. In addition, by culturing tonsil organoids, it is possible to overcome the physiological and evolutionary differences in humans, which are limitations of prior animal models.

As used herein, the term "tonsil" or "tonsillar" means a tonsil or a tonsil epithelium, which is a collection of lymph nodes, and includes pharyngeal tonsils (adenoids), tubal tonsils, throat tonsils (palatine tonsil) or lingual tonsils.

As used herein, the term "tonsil organoid" or "tonsillar organoid" refers to an organoid derived from tonsil-derived cells comprising tonsils or tonsil epithelia. The tonsil organoid may express the properties of the tonsil epithelium as it is. For example, it may express markers known to exhibit the characteristics of the tonsil epithelium through analysis methods such as chemotaxis analysis, qRT-PCR, cytokine analysis, and immunofluorescence analysis.

As used herein, the term "medium" or "media" refers to a medium that can support a growth, a survival and a differentiation of stem cells in vitro, and includes all conventional media suitable for the culture and differentiation of stem cells used in the art. Depending on the type of cell, the type of medium and culture conditions can be selected at the technical level in the art. The medium used for culture is specifically a cell culture minimum medium (CCMM), and generally comprises a carbon source, a nitrogen source, and a microelement component. The cell culture minimum medium comprises, for example, DMEM (Dulbecco's Modified Eagle's Medium), MEM (Minimal essential Medium), BME (Basal Medium Eagle), RPMI1640, F-10, F-12, α-MEM (a-Minimal essential Medium), GMEM (Glasgow's Minimal essential Medium), IMDM (Iscove's Modified Dulbecco's Medium), etc., but is not limited thereto. In addition, the medium may contain an antibiotic such as penicillin, streptomycin, gentamicin, or a mixture of two or more thereof.

As used herein, the term "TFM" refers to a Tonsil Formation Medium, and refers to a composition in which epidermal growth factor (EGF) is removed from human colonic organoid medium (HCM) or human prostate organoid medium (HPM).

In another embodiment, the human tonsillar organoid preparation medium comprises a HGF, a FGF10, a FGF2, a FGF basic, a PGE2, an A83-01, a Nicotinamide, a Noggin, a R-spondin1, an antibiotic-antifungal agent, a glutamax, a B27, a SB202190, a penicillin, a streptomycin, an EGF and a HEPES.

In another embodiment, the HGF is comprised in a concentration of 20 to 80 ng/ml in the total tonsil organoid preparation medium composition.

In one embodiment, a total concentration of the HGF in the medium composition is 10 to 100 ng/ml, 10 to 90 ng/ml, 10 to 80 ng/ml, 10 to 70 ng/ml, 10 to 60 ng/ml, 20 to 80 ng/ml, 20 to 70 ng/ml, 20 to 60 ng/ml, 30 to 80 ng/ml, 30 to 70 ng/ml, 30 to 60 ng/ml, 40 to 80 ng/ml, 40 to 70 ng/ml, or 40 to 50 ng/ml.

In another embodiment, a tonsil organoid prepared by the tonsillar organoid preparation medium expresses genes of a CD44, a Nerve growth factor receptor, a Cytokeratin 5, a Cytokeratin 13, a Integrin alpha 6, or an Epithelial cell adhesion molecule.

In another embodiment, a tonsil organoid prepared by the tonsillar organoid preparation medium expresses a toll-like receptor (TLR) 2, 3, 4, or 6.

In another embodiment, a tonsil organoid prepared by the tonsillar organoid preparation medium can be subcultured with more than two passages.

As used herein, the term "subculture" refers to replacing a culture vessel or dividing a cell group and culturing it in a method to culture successive generations for continuous long-term culturing cells, specifically organoids in a healthy state. One-time replacement of the culture vessel or culturing by dividing the cell groups is called the first passage. In the present invention, the passage may be used interchangeably with the generation.

In one embodiment, the tonsil organoid prepared by the tonsillar organoid preparation medium expresses the same markers and TLRs as actual human tonsil organoids, and it was confirmed that normal culture and differentiation were possible for about 50 days from the 1st generation to the 5th generation, that is, from the 1st passage to the 5th passage.

In another embodiment, the present invention provides a method for preparing a tonsil organoid comprising preparing a tonsillar organoid preparation medium additionally comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM), and culturing a tonsil organoid in the tonsillar organoid preparation medium.

In one embodiment, the size of the tonsil organoid may be 100 to 300, 150 to 300, 200 to 300, 100 to 250, 100 to 200, or 150 to 250 micrometers.

In another embodiment, the present invention provides a method for screening an anti-inflammatory agent comprising contacting an inflammatory substance to a tonsil organoid, contacting a test substance to the tonsil organoid treated with the inflammatory substance, and classifying the test substance as the anti-inflammatory agent when the cytokine expression level of the tonsil organoid in contact with the test substance is decreased compared to a control group.

The inflammatory substance is that inducing inflammation in the tonsil organoid, and may include any substance, molecule, element, compound, entity, or a combination thereof. For example, it may include a protein, a polypeptide, a small organic molecule, a polysaccharide, or a polynucleotide. It may also be a natural product, a synthetic compound or a chemical compound, or a combination of two or more substances. More specifically, it may be LPS, TNF-alpha or other allergens.

In the present invention, "cytokine" refers to a protein that plays an important role in a cell signal transduction. The release of the cytokine influences the behavior of cells around the cytokine. Specifically, the cytokines are immunomodulatory agents involved in autocrine signaling, paracrine signaling, and endocrine signaling. In addition, they are produced by a wide range of cells, including endothelial cells, fibroblasts and various stromal cells, as well as immune cells such as macrophages, B lymphocytes, T lymphocytes and mast cells. They play a role in regulating the balance between humoral and cell-based immune responses, and regulating the maturation, growth and response of particular cell groups.

The cytokines, which are treated with the inflammatory substance to measure the change in expression level, include IL-2 (interleukin-2), IL-8 (interleukin-8), TNFα (Tumor Necrosis Factor-a), IL-22 (interleukin-22), IL-6 (interleukin-6), IL-1β (interleukin-1β), IL-11 (interleukin-11), EGF (epidermal growth factor), OSM (oncostatin M), IL-10 (interleukin-10), IL-1α (interleukin-la), CXCL1 (Chemokine (C-X-C motif) ligand 1), CXCL8 (Chemokine (C-X-C motif) ligand 2), IL-1ra (interleukin-1ra), MIF (migration inhibition factor) or PAI-1 (plasminogen activator inhibitor 1).

The test substance is one expected to show an effect on the anti-inflammatory action of the tonsil organoid, for example, any substance, molecule, element, compound, entity, or a combination thereof. For example, it may include a protein, a polypeptide, a small organic molecule, a polysaccharide, or a polynucleotide. It may also be a natural product, a synthetic compound, a chemical compound, or a combination of two or more substances.

As used herein, the term "control group" refers to a tonsil organoid not treated with an anti-inflammatory test substance after treating with an inflammatory substance, and is used to compare the amount of cytokine secretion with the tonsil organoid treated with the anti-inflammatory test substance. The decrease of the cytokine secretion is proportional to the anti-inflammatory effect of the test substance. If the amount of cytokine secretion is decreased compared to the control, it can be determined that the test substance is an anti-inflammatory drug for the tonsil organoid.

A tonsillar organoid prepared by the tonsil organoid preparation medium according to one embodiment of the present invention can be subjected to stable long-term subculture, and it can show an immune response similar to that in the body by expressing the same markers and TLRs as in the actual tonsil epithelium.

### [Brief Description of Figures]

FIG. 1 is a result of culturing tonsil epithelial tissue after removing EGF from HCM or HPM.
FIG. 2 is a result of confirming whether long-term subcultures are possible depending on epithelial tissue markers and medium composition of the tonsil organoid.
FIG. 3 is a result confirming the similarity of the tonsil organoid structure with the tonsil epithelium.
FIG. 4 is a result confirming the change of the structure of tonsil organoid due to virus infections.
FIG.5 is a result of confirming the amount of cytokine secretion and TLR expression of the tonsil organoid.

### [EXAMPLES]

Hereinafter, preferred examples are presented to help the understanding of the present invention. However, the following examples are provided for easier understanding of the present invention, and the contents of the present invention are not limited by the following examples.

All statistical analyses were performed using GraphPad Prism 5. A quantitative statistical analysis was performed by unpaired two-samples t-test for comparison of two groups, and by one-way analysis of variance with Newman-Keuls multiple comparison test for comparison of multiple groups. The threshold for statistical significance was P <0.05. No statistical methods were used to predetermine the sample size. The sample size of the experiments was estimated using NIS imaging software. All details for statistical analysis and sample size are mean ± SEM.

### Example 1: Preparation tonsil organoid and Tonsil Formation Medium (TFM)

For preparing an organoid from human palatine tonsils, a conventional culture protocol using human colonic organoid medium (HCM) or human prostate organoid medium (HPM) was modified as follows.

A tonsil epithelial tissue is chopped with scissors and then washed using 1X phosphate buffer solution (PBS, Welgen). The minced tonsil epithelial tissue was enzymatically dissociated using Advanced DMEM/F12 (Gibco) containing 500 ug/ml collagenase type2 (Gibco) at 37°C for 2 hours.

The enzymatically dissociated human palatine cells were collected, pelleted, embedded in matrigel (Corning) and seeded in 48-well plates (SPL). The tonsil cells were grown with 1X antibiotic-agonist (Thermo Fisher Scientific), 1X Glutamax (Thermo Fisher Scientific), 1X B27 (Invitrogen), 10% R-spondin-1 (RSPO1) conditioned medium and growth factors containing Advanced DMEM / F12 (Gibco). An additional composition of the R-spondin-1 conditioned medium was as follows: FGF10 (50 ng/ml), FGF2 (= FGF basic, 20 ng/ml), PGE2 (10 µM), A83-01 (200 µM), SB202190 (=4-[4-(4-Fluorophenyl)-5-(4-pyridinyl)-1H-imidazol-2-yl]phenol, 10 µM), Nicotinamide (10 mM), Noggin (100 ng/ ml).

Neuregulin-1 (NRG1, Peprotech) was added only to the pharyngeal tonsil. After subculture, 10 uM Y-27632 (Tocris) was added to the culture medium for 2 days.

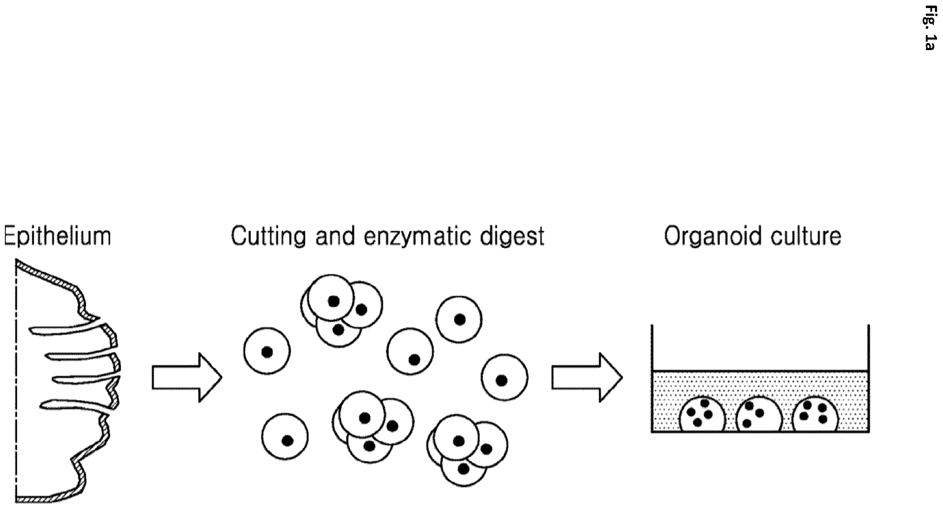

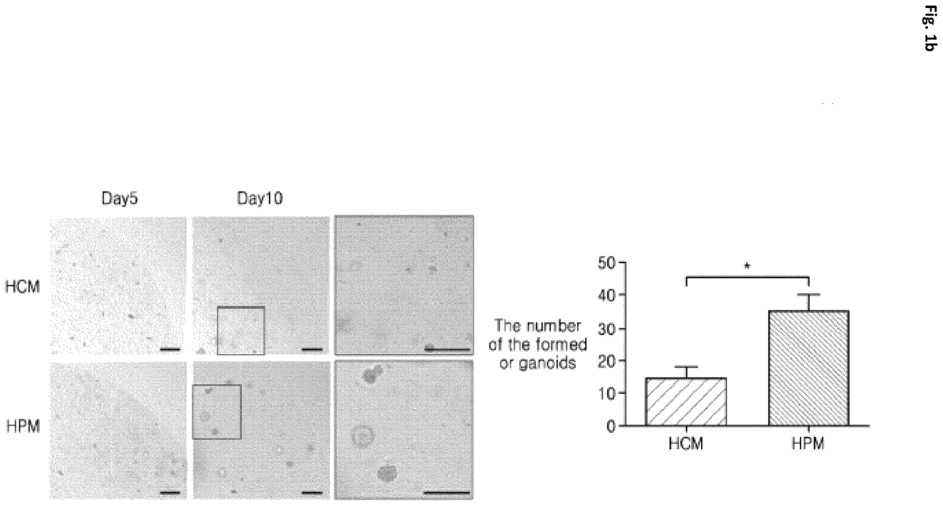

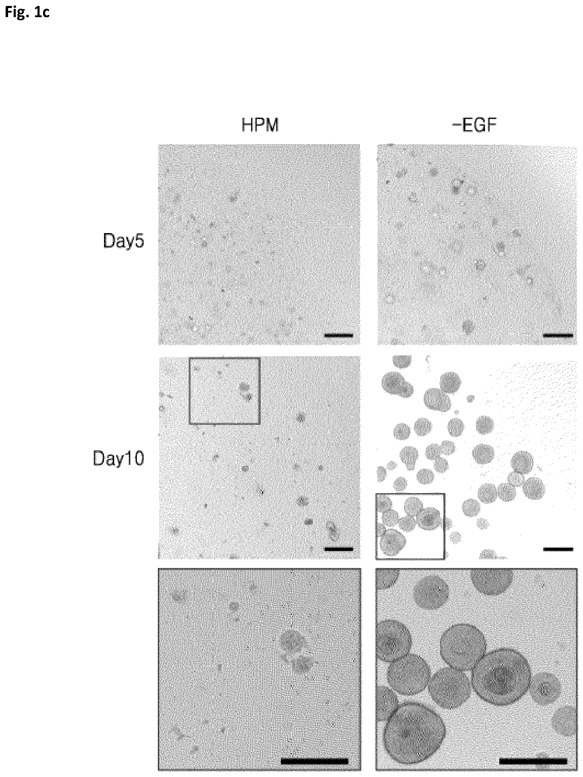

The enzymatically dissociated human palatine cells were suspended in a matrigel, and grown in the HCM or HPM comprising EGF, Noggin, FGF2, FGF10 and R-spondin-1, as shown in FIG 1A. As shown in FIG. 1B, palatine tonsil cells cultured in HPM on day 10 produced a higher number of 3D tonsil organoid compared to palatine tonsil cells cultured in HCM. To screen for the most productive medium composition, the dissociated tonsil cells were plated under various conditions prepared by removing or adding each component of HPM. As shown in FIG. 1C, the medium in which EGF was removed from HPM was most effective for culturing the tonsil organoid, and it was named tonsil forming medium (TFM).

### Example 2: Long-term subculture of tonsil organoid and identification of expression markers

Organoids of adult epithelial tissues such as stomach, intestine, endometrium, and liver can grow under subculture over several months. A tonsil organoid grown in TFM were cultured at a subcultivation ratio of 1:3 every 10 days, but it was not possible to subculture more than two generations of these organoids.

Therefore, in this example, in order to confirm a medium composition that allows the tonsil organoid to be stably subcultured for a long period of time, the tonsil organoids were cultured in various medium compositions and a qRT-PCR was performed.

The qRT-PCR was performed as follows. After the organoids were isolated from matrigel, washed with PBS, and spun down, an RNA isolation was performed using an RNA extraction kit (Bioneer) according to the protocol. The qPCR was performed on the samples using a TP950 Thermal Cycler Dice Real Time SysTEM III (TAKARA). Table 1 shows a list of primers used.

**[Table 1]**

| **Primer** | | **Sequence** |
|---|---|---|
| **KRT5** | Forward | GAGATCGCCACTTACCGCAA |
| | Reverse | GTAGCTTCCACTGCTACCTCC |
| **KRT13** | Forward | GGGACTCATCAGCAGCATCG |
| | Reverse | AGGGAAACCAATCATCTTGGC |
| **CD44** | Forward | TGGACAGGACAGGACCTCTT |
| | Reverse | AGGTCCTGCTTTCCTTCGTG |
| **NGFR** | Forward | ATCCCTGGCCGTTGGATTAC |
| | Reverse | GACAGGGATGAGGTTGTCGG |
| **ITGA6** | Forward | TGTGCTTGCTCTACCTGTCG |
| | Reverse | CGTGGGGTCAGCATCGTTAT |
| **TLR2** | Forward | CTGTGCTCTGTTCCTGCTGA |
| | Reverse | GATGTTCCTGCTGGGAGCTT |
| **TLR3** | Forward | GTCCCAAGCCTTCAACGACT |
| | Reverse | GTTTGCGTGTTTCCAGAGCC |
| **TLR4** | Forward | GGTGCCTCCATTTCAGCTCT |
| | Reverse | ACTGCCAGGTCTGAGCAATC |
| **TLR6** | Forward | TGGATGTGGCAGCTTTAGCA |
| | Reverse | AGAA TCAGGCCAGCCCTCT A |

In order to improve long-term subculture conditions, HGF and/or SB were removed from or added to the medium composition. The addition of HGF was done at a concentration of 50 ng/ml.

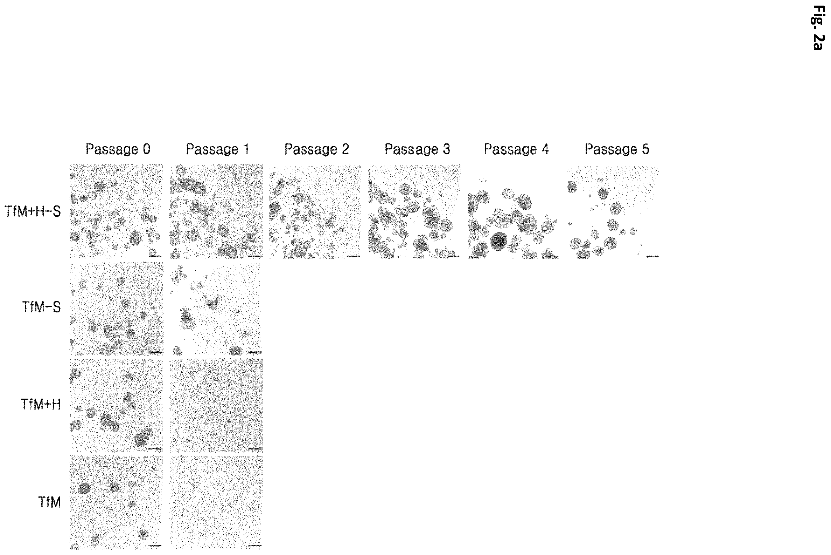

As shown in FIG. 2A, the organoids cultured in TFM for long-term passages showed a hierarchical structure such as tonsil epithelium, but growth was restricted in the second passage. In addition, removal of SB and addition of HGF to activate the p38 pathway did not change the formation and growth efficiency of the initially established organoids. However, the organoids cultured in TFM with HGF added and SB removed could be maintained for more than 5 passages or 60 days.

The TFM with HGF added and SB removed, which enabled the long-term subculture, was defined as a tonsillar organoid preparation medium (Tonsil Expansion Medium, TEM). An organoids cultured in the TEM were analyzed for gene expression patterns through a tonsil epithelial tissue markers such as a CD44, a Nerve growth factor receptor (NGFR), a Cytokeratin 5 (CK5), a Cytokeratin 13 (CK13), an Integrin alpha 6 (ITGA6) and an epithelial cell adhesion molecule (EpCAM). The mRNA levels of tonsil epithelial tissue markers of the organoids were monitored for 5 to 15 days, and compared with markers of actual human tonsil tissue epithelium.

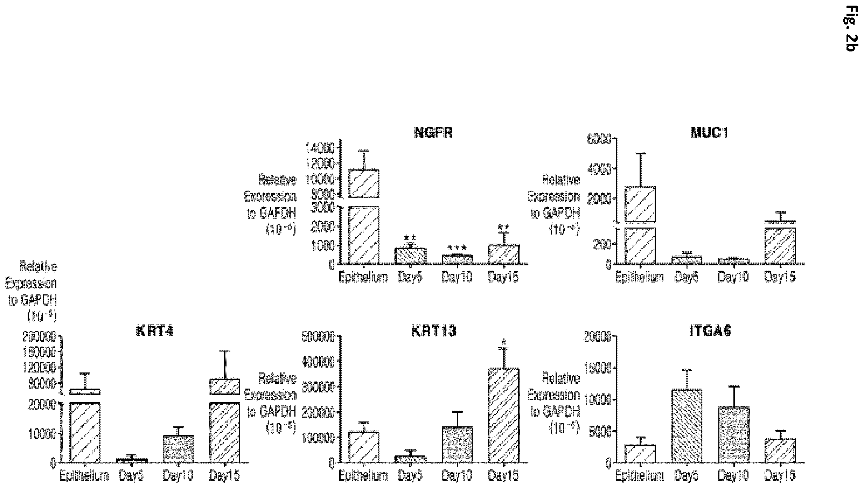

As shown in FIG. 2B, all of the tonsil epithelial tissue markers such as CD44, NGFR, CK5, CK13, ITGA6 and EpCAM were expressed in the TEM-grown organoids. The NGFR expression in the organoids was lower than in tonsil epithelium, but the EpCAM, the CD44, the KRT13, the KRT5 and the ITGA6 expressions were higher than in tonsil epithelium. In particular, the level of the cell differentiation marker CK13 increased with a longer culture period.

According to these examples, it was confirmed that the tonsil organoid cultured in the human tonsil expansion medium (TEM), defined as the TFM adding HGF and removing SB, can be subcultured for a long period of time.

### Example 3: Identification of structural features of tonsil organoid

A luminal surface of the palatine tonsil is covered with a typical non-keratinized stratified squamous epithelium arranged in layers on a basement membrane. Cells closer to the basement membrane have stronger self-renewal properties and have more cubic shapes, whereas cells near the surface have the property to differentiate into a flat shape.

In order to confirm the structural features of the tonsil organoid cultured in the TEM of Example 2, H&E staining and immunofluorescence were performed.

The H&E staining was performed as follows. The tonsil organoid was fixed in 4% paraformaldehyde (PFA, bio solution), then mixed with 2% agarose gel in a Cryo mold (10 × 10 × 5 mm, k4555, SungWon Medical Company) and solidified at room temperature. After that, a tissue processor (TP1020, Leica) was used for paraffin embedding at each step: 70 % EtOH for 1 hour, 80 % EtOH for 1 hour h, 90 % EtOH for 1 hour, 95 % EtOH for 1 hour, 100% EtOH twice for 1 hour, xylene twice for 1 hour, and paraffin twice for 2 hours in vacuum. Paraffin sections were cut to a thickness of 5 µm. And then, the paraffin section samples were deparaffinized three times in xylene for 3 minutes, and rehydrated in 100% EtOH for 3 minutes, 95% EtOH for 3 minutes, 90% EtOH for 3 minutes, 80% EtOH for 3 minutes and 70% EtOH for 3 minutes. They were rinsed three times in distilled water for 3 minutes, nuclei were stained in Harris Hematoxylin (SH3777, Cancer Diagnostics) for 5 minutes, then rinsed three times in distilled water for 3 minutes, discriminated in 1% acid alcohol for 1 second, then rinsed three times for 3 minutes in distilled water, cytoplasm was stained in Eosin Y (EM500G, Cancer Diagnostics) for 1 minute, and rinsed three times in distilled water for 3 minutes. The samples subjected to the above staining process were dehydrated with 70% EtOH for 3 minutes, 80% EtOH for 3 minutes, 90% EtOH for 3 minutes, 95% EtOH for 3 minutes, 100% EtOH twice for 3 minutes, and xylene twice for 3 minutes. Then, the samples are covered, stored at room temperature, and observed under a microscope.

The immunofluorescence was performed as follows. Tonsil tissue and organoid were fixed in 4% paraformaldehyde (PFA, biosolution). The organoid was mixed with 2% agarose gel in a Cryo mold (10 × 10 × 5 mm, k4555, SungWon Medical Company) and solidified at room temperature. A tissue processor (TP1020, Leica) was then used for paraffin embedding at each step. It was treated with 70 % EtOH for 1 hour, 80 % EtOH for 1 hour, 90 % EtOH for 1 hour, 95 % EtOH for 1 hour, 100% EtOH twice for 1 hour, xylene twice for 1 hour and paraffin twice for 2 hours in vacuum. Paraffin sections were cut to a thickness of 5 µm using microtome (RM2235, Leica). After that, the samples were immersed in xylene twice for 3 minutes, in xylene and 100% EtOH 1:1 for 3 minutes, and in 100% EtOH twice for 3 minutes. The paraffin sections were rehydrated with 95% EtOH for 3 minutes, 70% EtOH for 3 minutes, and 50% EtOH for 3 minutes, and then were subjected to antigen retrieval using sodium citrate buffer (10 mM sodium citrate with 0.05 % Tween 20, pH 6) in a 95°C water bath. The samples were washed twice for 5 minutes with 0.025 % Tx-100 in TBS for 5 minutes and blocked with blocking solution (10% NGS with 1% BSA in TBS) for 2 hours at room temperature. They were incubated overnight with a primary antibody diluted in TBS with 1% BSA on ice. They were washed with 0.025% Tx-100 in TBS and then incubated with a secondary antibody diluted in TBS with 1% BSA for 1 hour. After the samples were washed twice for 5 min with TBS, the samples were cultured with Hoechst solution diluted in PBS. They were washed twice using PBS. Slides were examined under a fluorescence microscope.

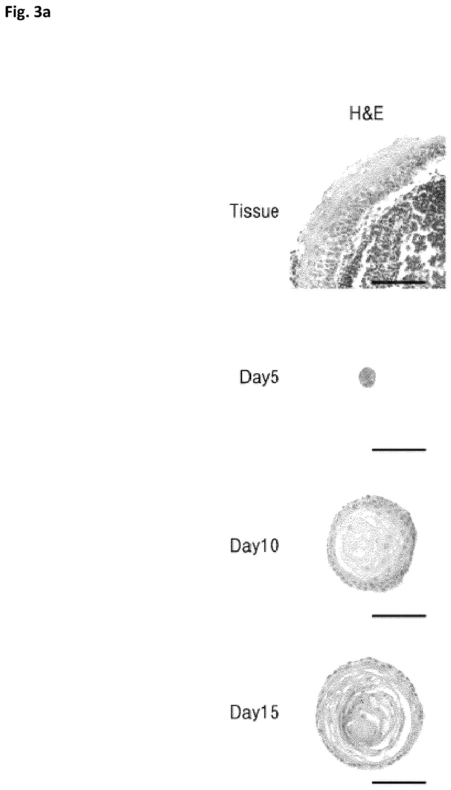

As shown in FIG. 3A, an organized multi-layered structure, similar to the stratified epithelium of tonsil tissue, was always observed in the H&E-stained cultured organoids.

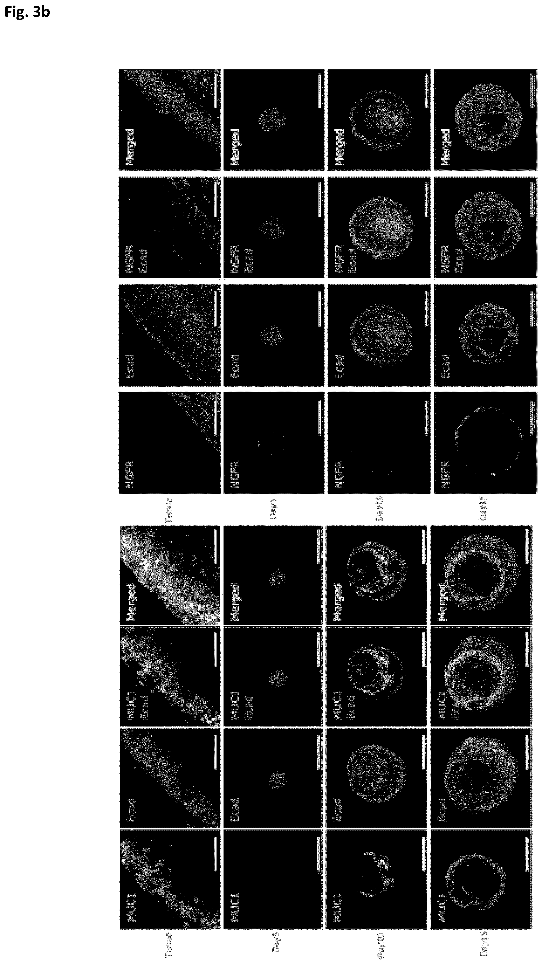

As shown in FIG. 3B, based on the epithelial marker e-cadherin, the outer layer of the tonsil organoid was stained with NGFR corresponding to the basal layer of the tonsil tissue. On the contrary, Muc1 was stained and detected in the inner layer of the tonsil organoid, whereas it ranged from the parabasal to upper layers of the tonsil tissue. This shows that the tonsil organoid structure is similar to the structure of the tonsil epithelium.

In order to test the migration of proliferating cells in the tonsil organoid, on day 10, the tonsil organoids were treated with BrdU for 2 days, and then doubled immunofluorescence staining was performed with Ki67, NGFR, CK5, CK4 and bromodeoxyuridine (BrdU) on day 15.

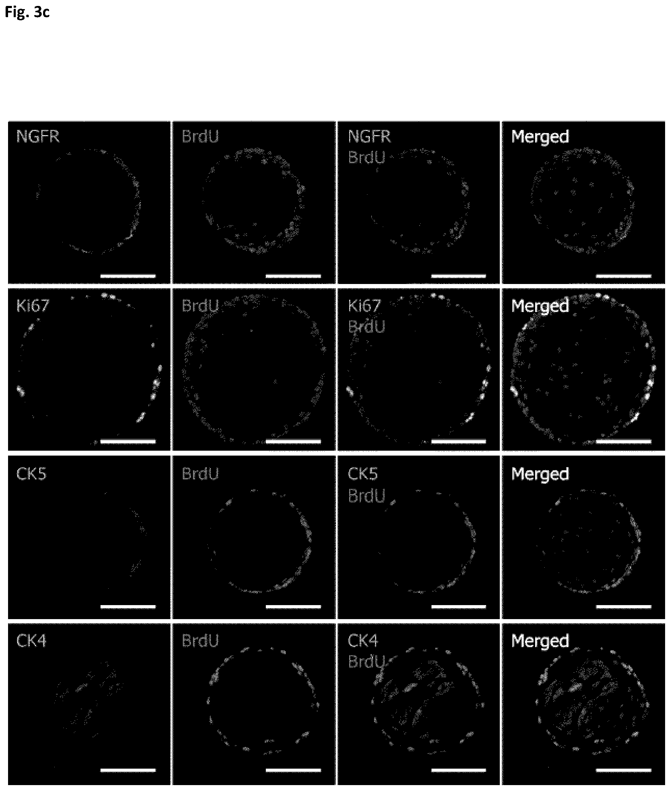

As shown in FIG. 3C, NGFR- and Ki67-stained cells were detected in the outer layer of the tonsil organoids, i.e., the region corresponding to the basal layer of tissue, whereas Muc1-stained cells were detected in the inner layer of the tonsil organoids, i.e., the region corresponding to the upper layer of the tissue. BrdU-stained cells were detected in several additional layers comprised in the basal layer. As a result, on the 10th day, it was confirmed that the BrdU-treated cells migrated toward the center of the tonsil organoids, indicating that differentiation was made in the inward direction.

### Example 4: Whether HPV16 E6/E7 Virus causes structural changes in tonsil organoids

In the palatine tonsils, we observed whether the HPV virus by head and neck cancer causes structural changes in the tonsil organoids.

An infection with lentiviral vectors was performed as follows. Briefly, the tonsil organoids cultured on day 5 were dissociated into single cells with 0.25% trypsin-EDTA (25200-072, GIBCO) in a 37°C water bath for 3 minutes, and neutralized with 10% FBS. During neutralization, 100 ul of cold liquid matrigel (354230, BD) was added to a 24-well plate and incubated at 37°C for 20 minutes to solidify the matrigel. 125 µl viral particles and 125 µl of 1×10⁵ single cell suspension in 2X tonsillar organoid culture medium were mixed in a 1.5 ml Eppendorf tube. 10 µM Y27632 (Y0503, Sigma Aldrich), 2.5 µM CHIR-99021 (SML1046, Sigma Aldrich) and 8 µg/ml polybrene (H9268, Sigma Aldrich) were added and mixed well by tapping. Then, a 250 µl mixture of the viral particles and the single cells was added to the solidified matrigel, and incubated at 37°C overnight. The next day (-16 hours), the cells were harvested, the virus-containing medium was removed, and cultured in a dome shape on a 48-well plate in a ratio of medium 1: matrigel 1. The cells were cultured in the tonsillar organoid medium supplemented with 10 uM Y-27632 (Y0503, Sigma Aldrich) for the first 3 days. Three days after infection, the transduced cells were selected with 0.125 ug/ml puromycin, and the selected organoids were cultured for 15 days.

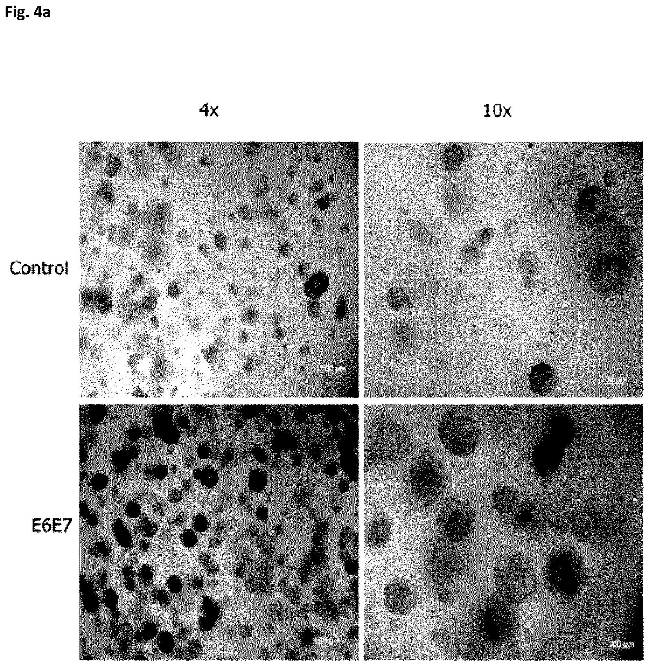

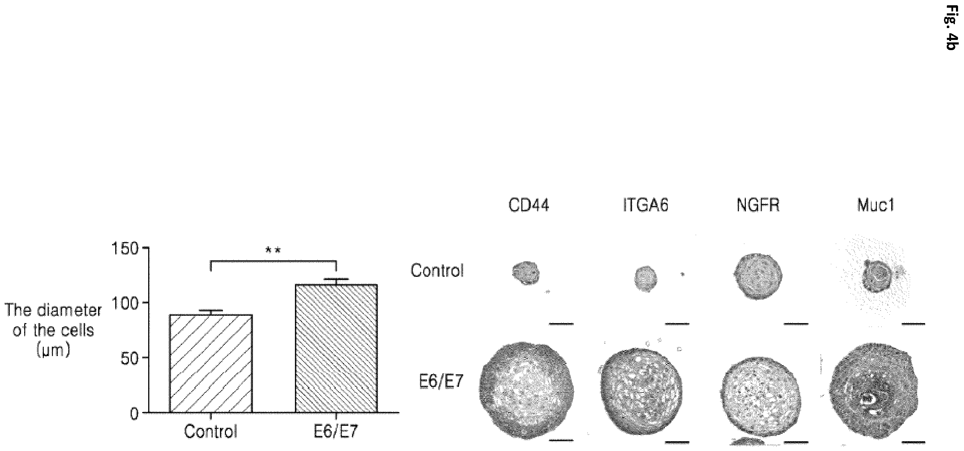

As shown in FIGS. 4A and 4B, it was confirmed that the E6/E7 transduced organoids had a larger diameter than the control group. In order to confirm how the E6/E7 transduced organoids change structurally, the fluorescent antibody method (immunocytochemistry, ICC) was performed using CD44, ITGA6, NGFR, and MUC1 antibodies. As a result, as shown in FIG. 4C, it was confirmed that cells forming the basal layer and the cells forming the layer just above the basal layer were more stained in the E6/E7 transduced organoids compared to the control group. As a result, it was confirmed that E6/E7 caused the structural change of the organoid.

### Example 5: Whether tonsil organoids release cytokines

In order to prove that the cytokine secretion function exists in the tonsil organoids of the present invention as in the tonsil epithelium, the relative secretion amounts of various cytokines released from the tonsil organoids were measured.

The analysis of the cytokines was performed as follows. This was performed using the Proteome Profiler Human Cytokine Array Kit (ARY005B, R&D SysTEMs) according to the manufacturer's protocol. Tonsillar organoid culture supernatants were prepared from untreated palatine tonsil organoids and palatine tonsil organoids treated with 10 ug/ml LPS. Pipette 2.0 mL of Array Buffer 4 was put into a 4-well multi-dish and the membrane was placed in the wells of the 4-well multi-dish. After incubating the membrane for 1 hour, a mixture of the organoid culture supernatant incubated for 1 hour, Array Buffer 4 and the Human Cytokine Array Detection Antibody Cocktail incubated for 1 hour was added to the 4-well multi-dish. Next, the 4-well multi-dish containing membranes and mixtures were incubated overnight at 2-8°C, and then the membranes were washed three times for 10 min with 1X wash buffer. Streptavidin-HRP diluted with 2 mL of Array Buffer 5 was added to the 4-well multi-dish, and incubated for 30 minutes. The membrane was then washed three times for 10 min with 1X wash buffer. The membrane was placed on a plastic sheet and 1 mL of Chemi reagent mixture was added to the membrane. Chemically treated membranes were detected with Chemi reagent mixtures using a LAS4000.

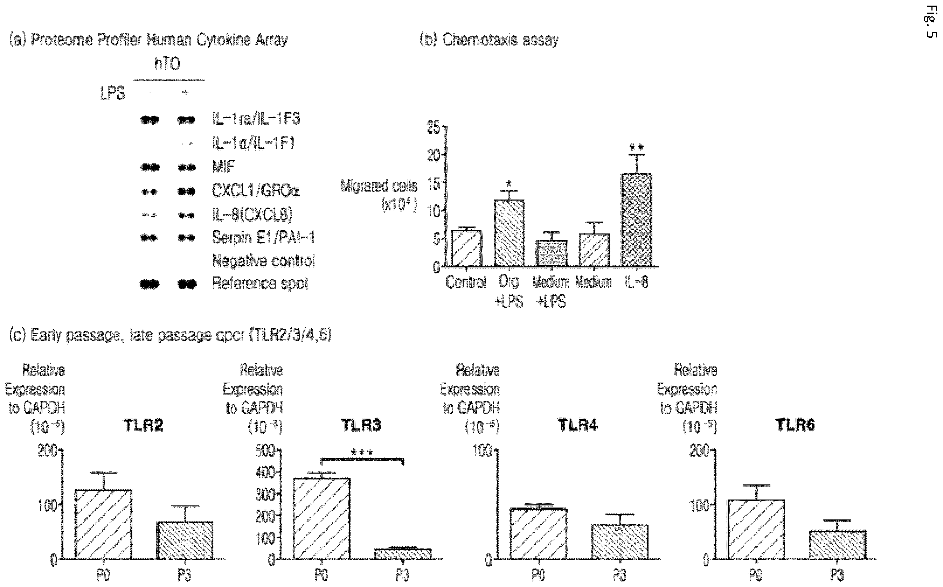

As shown in Figure 5A, LPS-stimulated tonsil organoids released more IL-1α, CXCL1 and CXCL8 compared to a control group, which were unstimulated tonsil organoids. It was confirmed that IL-1ra, MIF and PAI-1 were released lower in the stimulated tonsil organoids than in the control group.

### Example 6: Confirmation of neutrophil chemotaxis in tonsil organoids

In order to confirm the chemotaxis of neutrophil in the tonsil organoids, a chemotaxis analysis and a quantitative PCR were performed.

The chemotaxis analysis was performed using HL-60 cells with neutrophil-like properties as follows. The HL-60 cells were purchased from the Korean Cell Line Bank. They were cultured in RPMI1640 medium (25 mM HEPES and L-glutamine, Hyclone) supplemented with 10% fetal bovine serum (FBS, Gibco). The cultured cells were stored at 37°C in a humidified atmosphere containing 5% CO₂. For use in experiments, the HL-60 cells were differentiated into granulocytes by the addition of 1.3% dimethyl sulfoxide (DMSO, Duchefa) for 7 days.

As shown in FIG. 5B, dHL-60 cells (diffrenciated-HL cells, granulocytes) showed greater mobility in the organoid group treated with LPS than in other groups. These results confirmed that tonsil organoids released cytokines, and the released cytokines increased the migratory activity of the dHL-60 cells.

### Example 7: Confirmation of TLRs in tonsil organoids

In order to confirm that the same toll-like receptor (TLR) exists in the tonsil organoids as in the tonsil tissue, and pathogen signals are transmitted to lymphocytes, expression levels of TLR2, 3, 4, and 6 were analyzed by a qRT-PCR.

The qRT-PCR was performed as follows. After organoids were isolated from matrigel, washed with PBS, and spun down, RNA isolation was performed using an RNA extraction kit (Bioneer) according to the protocol. The qPCR was performed on the samples using a TP950 Thermal Cycler Dice Real Time SysTEM III (TAKARA). The Table 1 shows a list of primers used.

As shown in FIG. 5C, the expression levels of TLR2, 4 and 6 did not change in passage 0 compared to passage 3, but the expression level of TLR3 was increased in passage 3 compared to passage 0. As a result, it was confirmed that the tonsil organoids have TLR and release cytokines in response to immune stimulation like the tonsil epithelium.

The description of the present invention described above is for illustration, and a person skilled in the art to which the present invention pertains can understand that it can be easily modified into other forms without changing the technical idea or essential features of the present invention. Therefore, it should be understood that the embodiments described above are illustrative in all respects and not restrictive.

## Claims

1. A tonsillar organoid preparation medium further comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM).

2. The tonsillar organoid preparation medium according to claim 1, wherein it comprises HGF, FGF10, FGF2, FGF basic, PGE2, A83-01, Nicotinamide, Noggin, R-spondin1, antibiotic-antifungal agent, glutamax, B27, SB202190, penicillin, streptomycin, EGF and HEPES.

3. The tonsillar organoid preparation medium according to claim 2, wherein the HGF is comprised in a concentration of 20 to 80 ng/ml in the total tonsillar organoid preparation medium composition.

4. The tonsillar organoid preparation medium according to claim 1, wherein a tonsillar organoid prepared in the tonsillar organoid preparation medium expresses one or more genes selected from the group consisting of CD44, nerve growth factor receptor, cytokeratin 5, cytokeratin 13, Integrin alpha 6, and epithelial cell adhesion molecule.

5. The tonsillar organoid preparation medium according to claim 1, wherein a tonsillar organoid prepared in the tonsillar organoid preparation medium expresses at least one selected from the group consisting of toll-like receptor (TLR) 2, 3, 4, and 6.

6. The tonsillar organoid preparation medium according to claim 1, wherein a tonsil organoid prepared in the tonsillar organoid preparation medium can be subcultured at least second generations.

7. A method for preparing tonsillar organoid comprising:
preparing a tonsillar organoid preparation medium further comprising a Hepatocyte Growth Factor (HGF) and not comprising a 4-[4-(4-Fluorophenyl)-5-(4-pyridinyl)-1H-imidazol-2-yl]phenol in a Tonsil Formation Medium (TFM); and
culturing a tonsillar organoid in the tonsillar organoid preparation medium.

8. A method for screening an anti-inflammatory agent comprising:
contacting an inflammatory causing substance to a tonsil organoid prepared by the method according to claim 7;
measuring a cytokine level after contacting a test substance to the tonsil organoid contacted with the inflammatory causing substance; and
comparing the measured cytokine level with an inflammation level of a control group not contacted with the test substance.
